# EUROPEAN PATENT APPLICATION

(11) **EP 4 579 993 A2**
(43) Date of publication of application: **02.07.2025**
(21) Application number: 24222984.7
(22) Date of filing: 23.12.2024
(51) Int. Cl.: H02J 7/00, A61M 1/36, H02J 9/00

(54) **EXTERNAL ENERGY STORAGE POWER SUPPLY MANAGEMENT METHOD, SYSTEM, DEVICE AND STORAGE MEDIUM**

(30) Priority: 28.12.2023 CN 202311838903
(71) Applicant: ChinaBridge (Shenzen) Medical Technology Co., Ltd., Shenzhen, Guangdong 518102 (CN)
(72) Inventor: LI, Yijiang, Shenzhen Guangdong, 518102 (CN)
(74) Representative: Pfenning, Meinig & Partner mbB

(57) **Abstract**

An external energy storage power management method, system, device and storage medium for life support equipment, when it is detected that the power of the external energy storage power is insufficient to power the whole machine, the duty cycle of the charging power of the two batteries is reduced. When the duty cycle of the charging power of the two batteries is reduced to 0, the power of the two batteries is detected. When the power of one of the batteries is sufficient to power the equipment, it is immediately switched to the high-power battery to power the equipment, and at the same time, the external energy storage power continues to charge the low-power battery.

## Description

### CROSS REFERENCE TO RELATED APPLICATIONS

The present application claims priority to Chinese Patent Application No. 202311838903.2, filed December 28, 2023, the entire disclosure of which is incorporated herein by reference.

### TECHNICAL FIELD

The embodiments of the present invention relate to the technical field of life-support medical equipment, and in particular to an external energy storage power supply management method, system, device and storage medium.

### BACKGROUND

Extracorporeal membrane oxygenation (ECMO) is an emergency or life support device often used in ambulances, helicopters or other outdoor scenes.

ECMO equipment is used in outdoor scenarios, 220 V AC power is usually not available. It is generally powered by a vehicle power supply or other energy storage batteries. However, the external energy storage power supply may not have enough power. When the power required by the device is greater than the power that the external energy storage power supply can provide, the external energy storage power supply will cut off the power supply due to overload protection. Although the patent with publication number CN113541263A provides a method for automatically switching circuits between external power supply and battery power supply for a portable device, the device can only be powered by the mutual exchange of external power supply and battery, and the device needs to be turned on and off to achieve this, which obviously cannot provide help for the endurance of ECMO equipment. In addition, the patent with publication number CN116581846A provides a method and system for intelligently controlling battery charging, but when the power of the external energy storage power supply is lower than the power required by the device, the main frequency and power consumption of the electronic device need to be reduced, so it is not suitable for ECMO equipment.

ECMO equipment is a life-support device. Reducing the power consumption of the equipment will affect its performance and cause unpredictable effects on patients. If the power consumption of the equipment is not reduced or the minimum protection unit cannot continue to reduce the power consumption after the power consumption of part of the equipment has been reduced, the external energy storage power supply will be powered off due to overload protection, which is not conducive to the endurance of the equipment. Therefore, an external energy storage power supply management solution is needed to improve the power supply endurance of medical equipment such as ECMO equipment.

### SUMMARY

To this end, embodiments of the present invention provide an external energy storage power supply management method, system, device and storage medium to solve the technical problem that when the external energy storage power supply is insufficient in power, the power is cut off prematurely due to overload triggering protection, resulting in a short power supply endurance time of medical equipment.

In order to achieve the above objectives, the embodiments of the present invention provide the following technical solutions:
According to a first aspect of an embodiment of the present invention, an embodiment of the present application provides an external energy storage power supply management method, the method comprising:
Performing a first real-time monitoring of the voltage of the external energy storage power supply;
If the voltage of the external energy storage power supply does not meet the first preset condition, the external energy storage power supply is controlled to directly power the main device, and the first battery and the second battery are charged at the same time, and the voltage of the external energy storage power supply is continuously monitored in a first real-time manner;
If the voltage of the external energy storage power supply meets the first preset condition, lowering the first charging power duty cycle of the first battery and the second battery;
Performing a second real-time monitoring on the first charging power duty cycle of the first battery and the second battery;
If the first charging power duty ratio of the first battery and the second battery does not meet the second preset condition, continue to perform the first real-time monitoring of the external energy storage power supply voltage;
If the first charging power duty ratios of the first battery and the second battery meet the second preset condition, performing a third real-time monitoring on the power levels of the first battery and the second battery;
If the power levels of the first battery and the second battery meet a third preset condition, controlling the external energy storage power supply and the first battery and the second battery to be connected in parallel to supply power to the main device;
If the power levels of the first battery and the second battery do not meet the third preset condition, the battery with higher power level is selected and marked as the power supply battery, and the other battery is marked as the rechargeable battery;
The power supply battery is switched to supply power to the main device, and the external energy storage power supply is controlled to only charge the rechargeable battery.

Furthermore, the first preset condition is set as the voltage of the external energy storage power supply being lower than a first preset threshold value for a first continuous time period, the first time period being 500 milliseconds to 1000 milliseconds, and the first preset threshold being 90% to 95% of a normal value of the voltage of the external energy storage power supply.

Furthermore, the second preset condition is set as that the first charging power duty cycle of the first battery and the second battery is reduced to 0.

Further, the third preset condition is set as the first power value of the first battery and the second power value of the second battery both do not reach their respective corresponding second preset thresholds, and the second preset thresholds corresponding to the first battery and the second battery are 2%-5% of their own power respectively. Further, an external energy storage power supply management method also includes:
If the power levels of the first battery and the second battery do not meet the third preset condition, performing a fourth real-time monitoring on the power levels of the first battery and the second battery;
If the power levels of the first battery and the second battery meet a fourth preset condition, performing a fifth real-time monitoring on the power levels of the current power supply battery and the current charging battery;
If the power levels of the current power supply battery and the current charging battery do not meet the fifth preset condition, continue to perform fourth real-time monitoring on the power levels of the first battery and the second battery;
If the power levels of the current power supply battery and the current rechargeable battery meet the fifth preset condition, then reselect the battery with higher power level as the power supply battery and mark the other battery as the rechargeable battery;
Switching the updated power supply battery to power the main device, controlling the external energy storage power supply to charge only the updated rechargeable battery, and continuing to perform a fourth real-time monitoring of the power of the first battery and the second battery;
Among them, the fourth preset condition is set as one of the first power value of the first battery and the second power value of the second battery exceeds the corresponding third preset threshold value, and the third preset threshold values corresponding to the first battery and the second battery are respectively greater than or equal to 3%-8% of their own power; the fifth preset condition is set as the power value of the current power supply battery is less than the first preset ratio of the power value of the current charging battery, and the first preset ratio is greater than or equal to 92% and less than or equal to 97%.

Furthermore, an external energy storage power supply management method also includes:
If the power levels of the first battery and the second battery do not meet the fourth preset condition, performing a sixth real-time monitoring on the power levels of the current power supply battery and the current charging battery;
If the power levels of the current power supply battery and the current charging battery do not meet the sixth preset condition, continue to perform fourth real-time monitoring on the power levels of the first battery and the second battery;
If the power levels of the current power supply battery and the current rechargeable battery meet the sixth preset condition, then reselect the battery with higher power level as the power supply battery and mark the other battery as the rechargeable battery;
Switching the updated power supply battery to power the main device, controlling the external energy storage power supply to charge only the updated rechargeable battery, and continuing to perform a fourth real-time monitoring of the power of the first battery and the second battery;
The sixth preset condition is set as a second preset ratio that the power value of the current power supply battery is less than the power value of the current charging battery, and the second preset ratio is greater than or equal to 99% and less than or equal to 99.7%.

Furthermore, an external energy storage power supply management method also includes:
If the power levels of the first battery and the second battery do not meet the fourth preset condition, performing a seventh real-time monitoring on the voltage of the external energy storage power supply;
If the voltage of the external energy storage power supply does not meet the seventh preset condition, continuing to perform seventh real-time monitoring on the voltage of the external energy storage power supply;
If the voltage of the external energy storage power supply meets the seventh preset condition, lowering the second charging power duty cycle of the rechargeable battery;
Performing an eighth real-time monitoring on the second charging power duty cycle of the rechargeable battery;
If the second charging power duty cycle of the rechargeable battery does not meet the eighth preset condition, continuing to perform an eighth real-time monitoring on the second charging power duty cycle of the rechargeable battery;
If the second charging power duty cycle of the rechargeable battery meets the eighth preset condition, controlling the external energy storage power supply and the first battery and the second battery to be connected in parallel to supply power to the main device;
Among them, the seventh preset condition is set as the voltage of the external energy storage power supply being lower than the fourth preset threshold value for a second consecutive time period, the second time period is 100 milliseconds-200 milliseconds, and the fourth preset threshold value is 96%-98% of the normal value of the external energy storage power supply voltage; the eighth preset condition is set as the second charging power duty cycle of the rechargeable battery is reduced to 0.

According to a second aspect of an embodiment of the present invention, an embodiment of the present application provides an external energy storage power supply management system, the system is applied to a power management module, the system comprises: an external energy storage power supply voltage monitoring unit, a battery monitoring unit and a circuit control unit,
The external energy storage power supply voltage monitoring unit performs a first real-time monitoring on the external energy storage power supply voltage;
If the voltage of the external energy storage power supply does not meet the first preset condition, the circuit control unit controls the external energy storage power supply to directly power the main device, and charges the first battery and the second battery at the same time, and the external energy storage power supply voltage monitoring unit continues to perform first real-time monitoring on the voltage of the external energy storage power supply;
If the voltage of the external energy storage power supply meets the first preset condition, the circuit control unit lowers the first charging power duty cycle of the first battery and the second battery;
The battery monitoring unit performs a second real-time monitoring on the first charging power duty cycle of the first battery and the second battery;
If the first charging power duty ratio of the first battery and the second battery does not meet the second preset condition, the battery monitoring unit continues to perform first real-time monitoring on the voltage of the external energy storage power supply;
If the first charging power duty ratios of the first battery and the second battery meet the second preset condition, the battery monitoring unit performs a third real-time monitoring on the power levels of the first battery and the second battery;
If the power levels of the first battery and the second battery meet the third preset condition, the circuit control unit controls the external energy storage power supply and the first battery and the second battery to be connected in parallel to supply power to the main device;
If the power levels of the first battery and the second battery do not meet the third preset condition, the circuit control unit selects the battery with higher power level as the power supply battery and marks the other battery as the rechargeable battery; switches the power supply battery to power the main device, and controls the external energy storage power supply to only charge the rechargeable battery.

According to a third aspect of an embodiment of the present invention, there is provided an external energy storage power supply management device, the device comprising: a processor and a memory;
The memory is used to store one or more program instructions;
The processor is used to run one or more program instructions to execute the steps of an external energy storage power supply management method as described in any of the above items.
aspect of an embodiment of the present invention, a computer-readable storage medium is provided, on which a computer program is stored. When the computer program is executed by a processor, the steps of an external energy storage power supply management method as described in any one of the above items are implemented.

Compared with the prior art, the embodiment of the present application provides an external energy storage power management method, system, device and storage medium. For life support equipment, when it is detected that the power of the external energy storage power is insufficient to power the whole machine, the duty cycle of the charging power of the two batteries is reduced; when the duty cycle of the charging power of the two batteries is reduced to 0, the power of the two batteries is detected, and when the power of one of the batteries is sufficient to power the equipment, it is immediately switched to the high-power battery to power the equipment, and at the same time, the external energy storage power continues to charge the low-power battery; when the power of both batteries is not sufficient to power the equipment, the circuit is directly switched to the external energy storage power supply and the two batteries in parallel to power the equipment. In this way, when the power of the external energy storage power supply is insufficient, the external energy storage power supply can be prevented from triggering the overload protection too early and cutting off the power , and the external energy storage power supply and the two batteries can be used as much as possible to continue to power the equipment, thereby improving the battery life of the ECMO equipment and better protecting the life safety of the patient.

### BRIEF DESCRIPTION OF THE DRAWINGS

In order to more clearly illustrate the implementation methods of the present invention and the technical solutions thereof, the following briefly introduces the drawings required for the implementation methods or the description of the prior art. Obviously, the drawings in the following description are only exemplary, and for ordinary technicians in this field, other implementation drawings can be derived from the provided drawings without creative work.

The structures, proportions, sizes, etc. illustrated in this specification are only used to match the contents disclosed in the specification so as to facilitate understanding and reading by persons familiar with the technology. They are not used to limit the conditions under which the present invention can be implemented, and therefore have no substantial technical significance. Any structural modification, change in proportion or adjustment of size shall still fall within the scope of the technical contents disclosed in the present invention without affecting the effects and purposes that can be achieved by the present invention.
FIG. 1 is a schematic diagram of the logical structure of an external energy storage power management system provided by an embodiment of the present invention;
FIG. 2 is a schematic diagram of a power management circuit for implementing the corresponding functions of a power management module provided by an embodiment of the present invention;
FIG. 3 is a schematic diagram of a flow chart of an external energy storage power supply management method provided by an embodiment of the present invention;
FIG. 4 is a schematic diagram of a circuit principle for controlling an external energy storage power supply to directly power a main device and simultaneously charge a first battery and a second battery in an external energy storage power supply management method provided by an embodiment of the present invention;
FIG. 5 is a schematic diagram of the circuit principle of switching the power supply battery to power the main device and controlling the external energy storage power supply to only charge the rechargeable battery in an external energy storage power supply management method provided by an embodiment of the present invention;
FIG. 6 is a schematic diagram of a circuit principle for controlling an external energy storage power supply and a first battery and a second battery to be connected in parallel to supply power to a main device in an external energy storage power supply management method provided by an embodiment of the present invention;
FIG. 7 is a flow chart of another external energy storage power supply management method provided by an embodiment of the present invention; and

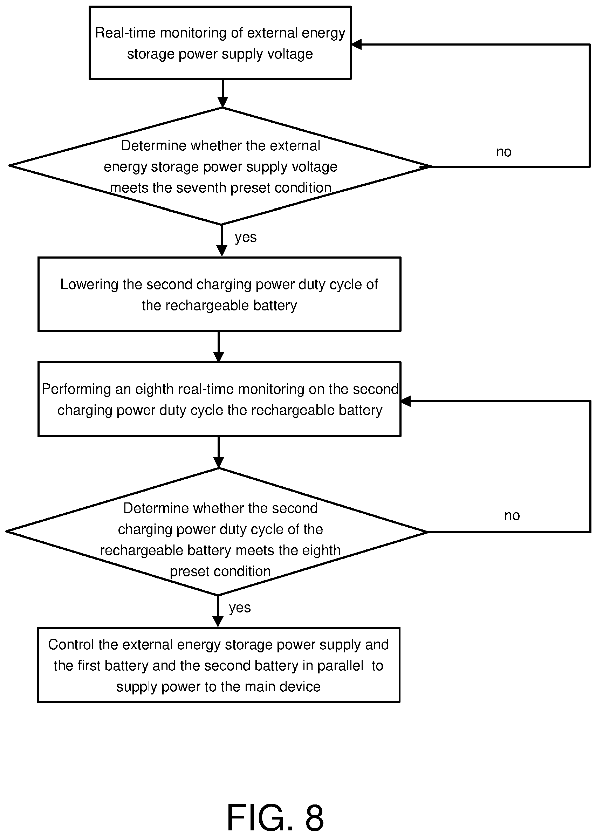

FIG. 8 is a flow chart of another external energy storage power supply management method provided in an embodiment of the present invention.

### DETAILED DESCRIPTION

The following is a description of the implementation of the present invention by specific embodiments. People familiar with the art can easily understand other advantages and effects of the present invention from the contents disclosed in this specification. The described embodiments are some of the embodiments of the present invention, not all of the embodiments. Based on the embodiments of the present invention, all other embodiments obtained by ordinary technicians in this field without creative work are within the scope of protection of the present invention.

The purpose of this application is to monitor and manage power supplies for high-life support medical equipment to prevent premature power outages due to overload protection when the external energy storage power supply is insufficient, thereby extending the power supply life of the medical equipment as much as possible.

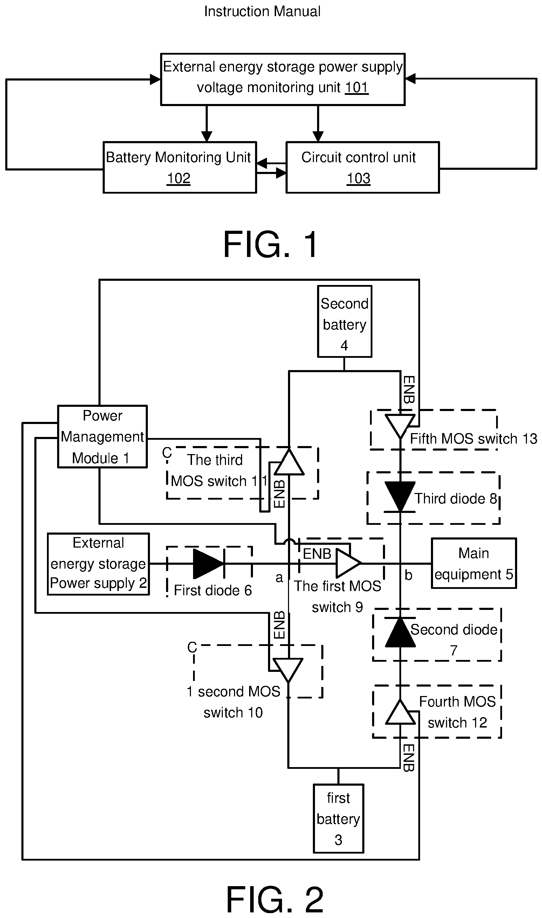

In order to solve the above technical problems, as shown in FIG. 1, an embodiment of the present application provides an external energy storage power management system, which is applied to a power management module 1.

In the embodiment of the present invention, the power management module 1 includes an MCU (Microcontroller Unit), whose main functions are to monitor the voltage of the external energy storage power supply, monitor the battery power and control the switch circuit.

Specifically, an external energy storage power supply management system provided in an embodiment of the present application includes: an external energy storage power supply voltage monitoring unit 101, a battery monitoring unit 102 and a circuit control unit 103.

With reference to FIG. 2, an external energy storage power management circuit for realizing the above-mentioned specific functions of the power management module 1 provided in an embodiment of the present invention is specifically as follows: the external energy storage power supply 2 is electrically connected to the main device 5, forming a first power supply circuit for directly supplying power to the main device 5 through the external energy storage power supply 2. A first node a is formed in the first power supply circuit at a position close to the external energy storage power supply 2, and the first power supply circuit is electrically connected to the first battery 3 through the first node a, so that a first charging circuit for charging the first battery 3 is formed between the external energy storage power supply 2 and the first battery 3. The first power supply circuit is electrically connected to the second battery 4 through the first node a, so that a second charging circuit for charging the second battery 4 is formed between the external energy storage power supply 2 and the second battery 4.

In the embodiment of the present invention, for example, the external energy storage power supply 2 can use the 12V DC output of a fully charged Juos 750W, 500,000 mAh mobile power supply to power the main device 5. The external energy storage power supply 2 can also use the 24V DC output of an ambulance onboard power supply to power the main device 5.

In addition, a second node b is formed in the first power supply circuit at a position close to the main device 5, and the first battery 3 is electrically connected to the second node b, so that a second power supply circuit for supplying power to the main device 5 through the first battery 3 is formed between the first battery 3 and the main device 5. The second battery 4 is electrically connected to the second node b, so that a third power supply circuit for supplying power to the main device 5 through the second battery 4 is formed between the second battery 4 and the main device 5.

Furthermore, the above-mentioned external energy storage power management circuit provided by the embodiment of the present invention includes: 5 MOS switches, which are respectively a first MOS switch 9, a second MOS switch 10, a third MOS switch 11, a fourth MOS switch 12 and a fifth MOS switch 13. The first MOS switch 9 is arranged between the first node a and the second node b, and the second MOS switch 10 is arranged between the first battery 3 and the first node a. The third MOS switch 11 is arranged between the second battery 4 and the first node a. The fourth MOS switch 12 is arranged between the first battery 3 and the second node b. The fifth MOS switch 13 is arranged between the second battery 4 and the second node b.

Furthermore, the gates of the first MOS switch 9, the second MOS switch 10, the third MOS switch 11, the fourth MOS switch 12 and the fifth MOS switch 13 respectively form ENB terminals, and the control signal output terminal of the power management module 1 is respectively connected to the ENB terminals of the first MOS switch 9, the second MOS switch 10, the third MOS switch 11, the fourth MOS switch 12 and the fifth MOS switch 13. In this way, the power management module 1 can control the on and off of the first MOS switch 9, the second MOS switch 10, the third MOS switch 11, the fourth MOS switch 12 and the fifth MOS switch 13 through the control signal. For example, the power management module 1 controls the one of the first MOS switch 9, the second MOS switch 10, the third MOS switch 11, the fourth MOS switch 12 and the fifth MOS switch 13 through the high level signal, and controls the off of the first MOS switch 9, the second MOS switch 10, the third MOS switch 11, the fourth MOS switch 12 and the fifth MOS switch 13 through the low level signal.

In addition, the above-mentioned external energy storage power supply management circuit provided by the embodiment of the present invention further includes: 3 diodes, which are respectively a first diode 6, a second diode 7 and a third diode 8. The first diode 6 is arranged between the external energy storage power supply 2 and the first node a, the second diode 7 is arranged between the fourth MOS switch 12 and the second node b, and the third diode 8 is arranged between the fifth MOS switch 13 and the second node b.

Furthermore, the external energy storage power supply voltage monitoring unit 101 performs a first real-time monitoring of the external energy storage power supply voltage; if the external energy storage power supply voltage does not meet the first preset condition, the circuit control unit 103 controls the external energy storage power supply 2 to directly power the main device 5, and charges the first battery 3 and the second battery 4 at the same time, and the external energy storage power supply voltage monitoring unit 101 continues to perform a first real-time monitoring of the external energy storage power supply voltage; if the external energy storage power supply voltage meets the first preset condition, the circuit control unit 103 lowers the first charging power duty cycle of the first battery 3 and the second battery 4.

The battery monitoring unit 102 performs a second real-time monitoring of the first charging power duty cycle of the first battery 3 and the second battery 4; if the first charging power duty cycle of the first battery 3 and the second battery 4 does not meet the second preset condition, the battery monitoring unit 102 continues to perform a first real-time monitoring of the voltage of the external energy storage power supply; if the first charging power duty cycle of the first battery 3 and the second battery 4 meets the second preset condition, the battery monitoring unit 102 performs a third real-time monitoring of the power levels of the first battery 3 and the second battery 4; if the power levels of the first battery 3 and the second battery 4 meet the third preset condition, the circuit control unit 103 controls the external energy storage power supply 2 and the first battery 3 and the second battery 4 to be connected in parallel to supply power to the main device 5; if the power levels of the first battery 3 and the second battery 4 do not meet the third preset condition, the circuit control unit 103 selects a battery with a higher power level and marks it as a power supply battery, and marks the other battery as a rechargeable battery; switches the power supply battery to supply power to the main device, and controls the external energy storage power supply 2 to charge only the rechargeable battery.

Compared with the prior art, the embodiment of the present application provides an external energy storage power management system. For life support equipment, when it is detected that the power of the external energy storage power is insufficient to power the entire device, the duty cycle of the charging power of the two batteries is reduced; when the duty cycle of the charging power of the two batteries is reduced to 0, the power of the two batteries is detected, and when the power of one of the batteries is sufficient to power the device, it is immediately switched to the high-power battery to power the device, and at the same time, the external energy storage power continues to charge the low-power battery; when the power of both batteries is not sufficient to power the device, the circuit is directly switched to the external energy storage power supply and the two batteries in parallel to power the device. In this way, when the external energy storage power supply is insufficient in power, the external energy storage power supply can be prevented from triggering the overload protection too early and cutting off the power , and the external energy storage power supply and the two batteries can be used as much as possible to continue to power the device, thereby improving the battery life of the ECMO device and better protecting the patient's life safety.

Corresponding to the external energy storage power management system disclosed above, the embodiment of the present invention further discloses an external energy storage power management method. The following describes in detail an external energy storage power management method disclosed in the embodiment of the present invention in combination with the external energy storage power management system described above.

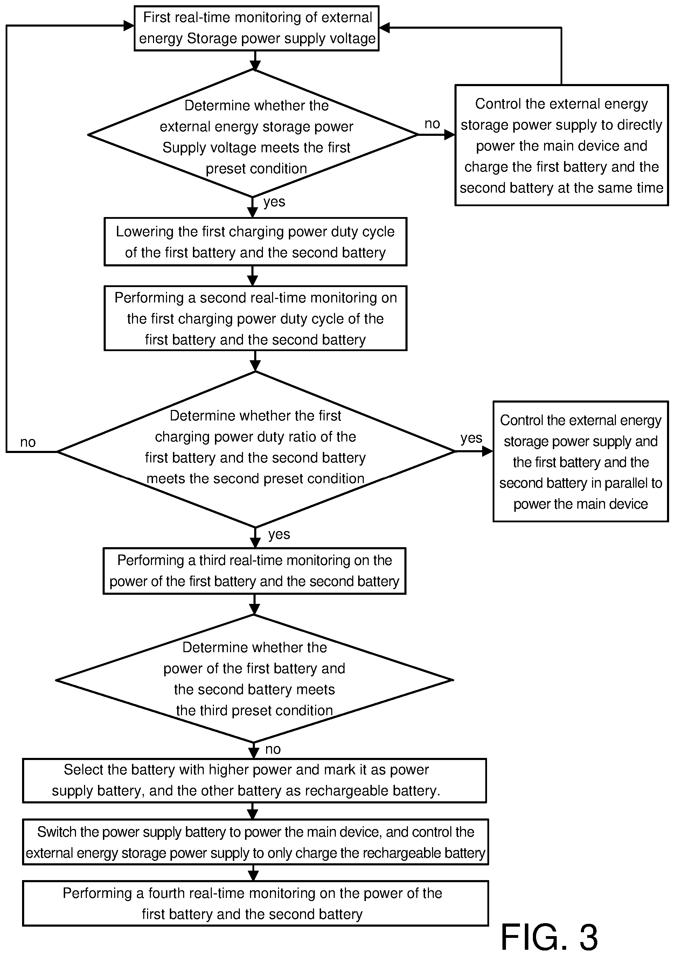

As shown in FIG. 3, the specific steps of an external energy storage power supply management method provided in an embodiment of the present application are described in detail below.

An external energy storage power supply management method provided in an embodiment of the present application is applied to a power management module 1. The power management module 1 has an MCU (Microcontroller Unit), whose main functions are to monitor the voltage of the external energy storage power supply, monitor the battery power and control the switch circuit. Specifically, the MCU implements the above functions through an external energy storage power supply voltage monitoring unit 101, a battery monitoring unit 102 and a circuit control unit 103.

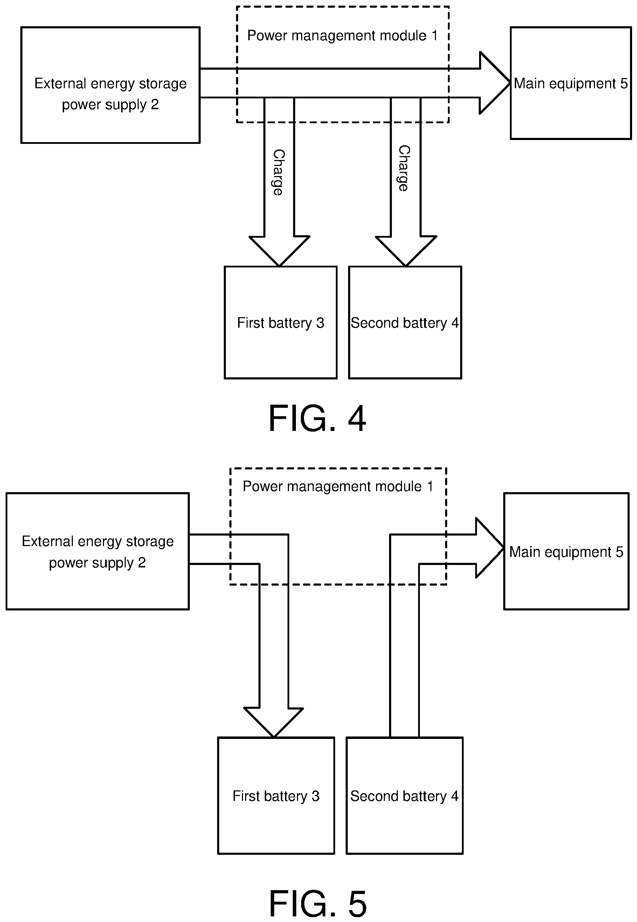

In the embodiment of the present invention, the external energy storage power supply voltage monitoring unit 101 monitors the external energy storage power supply voltage input in real time through ADC analog-to-digital conversion, and the sampling period is 50-200 milliseconds. When the power of the external energy storage power supply is sufficient to power the entire main device 5, an external energy storage power supply management circuit operates in accordance with the first power supply mode, as shown in FIG4, the external energy storage power supply 2 directly powers the main device 5 and charges the first battery 3 and the second battery 4. At this time, the output power of the external energy storage power supply 2 is equal to the power supply power of the main device 5 plus the charging power of the first battery 3 and the second battery 4.

Specifically, the external energy storage power supply voltage monitoring unit 101 performs a first real-time monitoring on the external energy storage power supply voltage. If the external energy storage power supply voltage does not meet the first preset condition, the circuit control unit 103 controls the external energy storage power supply 2 to directly supply power to the main device 5, and charges the first battery 3 and the second battery 4 at the same time, and the external energy storage power supply voltage monitoring unit 101 continues to perform a first real-time monitoring on the external energy storage power supply voltage.

When the voltage of the external energy storage power supply does not meet the first preset condition, the circuit control unit 103 controls the first MOS switch 9, the second MOS switch 10 and the third MOS switch 11 to be turned on, and the fourth MOS switch 12 and the fifth MOS switch 13 to be turned off. At this time, the external energy storage power supply 2 directly supplies power to the main device 5, and the external energy storage power supply 2 charges both the first battery 3 and the second battery 4 at the same time.

Further, in the embodiment of the present invention, the external energy storage power supply voltage monitoring unit 101 performs a first real-time monitoring of the external energy storage power supply voltage using a first preset condition, that is, the first preset condition is used to determine whether the power of the external energy storage power supply is sufficient to supply power to the entire main device 5. The first preset condition is set as the external energy storage power supply voltage being lower than a first preset threshold value for a continuous first time period, the first time period being 500 milliseconds to 1000 milliseconds, and the first preset threshold being 90% to 95% of a normal value of the external energy storage power supply voltage.

When in the first power supply mode, when it is found through the above-mentioned first real-time monitoring that the power of the external energy storage power supply is insufficient to supply power to the entire main device 5, the external energy storage power supply 2 still directly supplies power to the main device 5, and reduces the power duty cycle of charging the two batteries to adjust the power, thereby ensuring that the external energy storage power supply 2 continues to supply power to the main device 5 normally.

Specifically, if the voltage of the external energy storage power supply meets the first preset condition, the circuit control unit 103 lowers the first charging power duty cycle of the first battery 3 and the second battery 4.

Continuing, when the first charging power duty cycle of the first battery 3 and the second battery 4 is reduced to 0, and the first battery 3 or the second battery 4 has a certain amount of power, the working mode of an external energy storage power supply management circuit is switched to the second power supply mode, as shown in FIG5 , the main device 5 is powered by the battery with a high power, and the external energy storage power supply 2 charges the battery with a low power. At this time, because the external energy storage power supply 2 only needs to charge one battery, the power is only 25W, and the overload protection will not be triggered prematurely due to overcurrent.

Specifically, the battery monitoring unit 102 performs second real-time monitoring on the first charging power duty cycle of the first battery 3 and the second battery 4.

In the embodiment of the present invention, the second real-time monitoring is implemented by using the second preset condition, that is, the second preset condition is set as the first charging power duty cycle of the first battery and the second battery is reduced to 0.

If the first charging power duty ratio of the first battery 3 and the second battery 4 does not satisfy the second preset condition, the battery monitoring unit 102 continues to perform the first real-time monitoring on the voltage of the external energy storage power supply.

If the first charging power duty ratios of the first battery 3 and the second battery 4 meet the second preset condition, the battery monitoring unit 102 performs a third real-time monitoring on the power levels of the first battery 3 and the second battery 4.

In the embodiment of the present invention, specifically, the third real-time monitoring is realized by utilizing the third preset condition, and the third preset condition is set as that the first power value of the first battery 3 and the second power value of the second battery 4 do not reach their respective corresponding second preset thresholds, and the second preset thresholds corresponding to the first battery 3 and the second battery 4 are 2%-5% of their own power respectively.

If the power levels of the first battery 3 and the second battery 4 do not meet the third preset condition, the circuit control unit 103 selects the battery with higher power level as the power supply battery and marks the other battery as the rechargeable battery; switches the power supply battery to power the main device, and controls the external energy storage power supply 2 to only charge the rechargeable battery.

As described above, when the power of the first battery 3 and the second battery 4 does not meet the third preset condition, if the power of the first battery 3 is higher, the first battery 3 is marked as a power supply battery, and the second battery 4 is marked as a charging battery. In conjunction with FIG. 2, at this time, the circuit control unit 103 controls the third MOS switch 11 and the fourth MOS switch 12 to be turned on, and the first MOS switch 9, the second MOS switch 10 and the fifth MOS switch 13 are turned off. At this time, the first battery 3 is switched to supply power to the main device 5, and the external energy storage power supply 2 only charges the second battery 4.

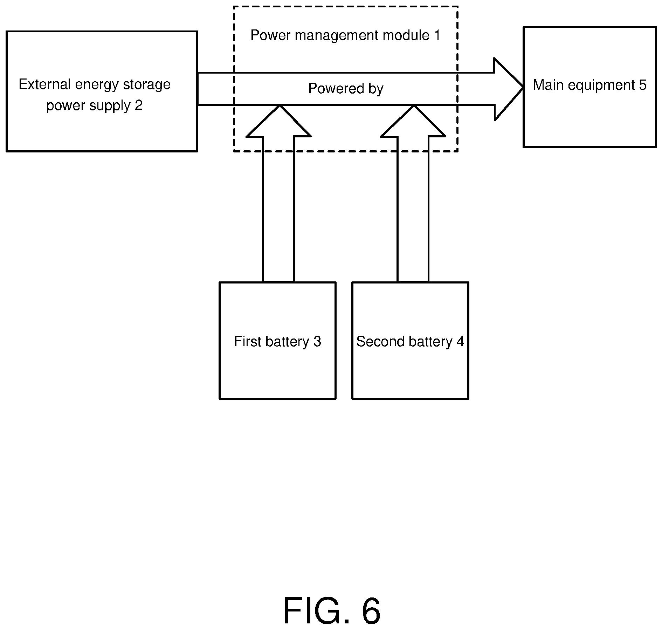

If the power of the first battery 3 and the second battery 4 meets the third preset condition, the circuit control unit 103 controls the external energy storage power supply 2 and the first battery 3 and the second battery 4 to be connected in parallel to supply power to the main device 5. That is, the working mode of an external energy storage power supply management circuit is switched to the third power supply mode, as shown in FIG. 6, the main device 5 is jointly powered by the external energy storage power supply 2, the first battery 3 and the second battery 4.

When the power levels of the first battery 3 and the second battery 4 meet the third preset condition, the circuit control unit 103 controls the first MOS switch 9, the fourth MOS switch 12 and the fifth MOS switch 13 to be turned on, and the second MOS switch 10 and the third MOS switch 11 to be turned off. At this time, the external energy storage power supply 2, the first battery 3 and the second battery 4 are connected in parallel to supply power to the main device 5.

Compared with the prior art, the external energy storage power management method provided in the embodiment of the present application is for life support equipment. When it is detected that the power of the external energy storage power is not enough to power the whole machine, the duty cycle of the charging power of the two batteries is reduced; when the duty cycle of the charging power of the two batteries is reduced to 0, the power of the two batteries is detected, and when the power of one of the batteries is sufficient to power the equipment, it is immediately switched to the high-power battery to power the equipment, and at the same time, the external energy storage power continues to charge the low-power battery; when the power of both batteries is not sufficient to power the equipment, the circuit is directly switched to the external energy storage power supply and the two batteries in parallel to power the equipment. In this way, when the power of the external energy storage power supply is insufficient, the external energy storage power supply can be prevented from triggering the overload protection too early and cutting off the power , and the external energy storage power supply and the two batteries can be used as much as possible to continue to power the equipment, thereby improving the battery life of the ECMO equipment and better protecting the life safety of the patient.

In an embodiment of the present invention, after the working mode of an external energy storage power supply management circuit is switched to the second power supply mode, in this mode, it is necessary to keep the power balance of two batteries.

Further, if the power levels of the first battery 3 and the second battery 4 do not satisfy the third preset condition, a fourth real-time monitoring is performed on the power levels of the first battery 3 and the second battery 4.

Referring to FIG. 3, preferably, when the power levels of the first battery 3 and the second battery 4 do not meet the third preset condition, the circuit control unit 103 selects the battery with higher power level as the power supply battery and marks the other battery as the rechargeable battery; after switching the power supply battery to power the main device and controlling the external energy storage power supply 2 to only charge the rechargeable battery, a fourth real-time monitoring of the power levels of the first battery 3 and the second battery 4 is performed.

Specifically, in the embodiment of the present invention, the fourth real-time monitoring is implemented by utilizing the fourth preset condition, and the fourth preset condition is set as one of the first power value of the first battery 3 and the second power value of the second battery 4 exceeds their respective corresponding third preset thresholds, and the third preset thresholds corresponding to the first battery 3 and the second battery 4 are 3%-8% of their own power respectively.

If the power levels of the first battery 3 and the second battery 4 meet the fourth preset condition, a fifth real-time monitoring is performed on the power levels of the current power supply battery and the current charging battery.

Specifically, in an embodiment of the present invention, the fifth real-time monitoring is implemented using the fifth preset condition, and the fifth preset condition is set to a first preset ratio in which the power value of the current power supply battery is less than the power value of the current charging battery, and the first preset ratio is greater than or equal to 92% and less than or equal to 97%.

If the power levels of the current power supply battery and the current charging battery do not meet the fifth preset condition, the fourth real-time monitoring of the power levels of the first battery 3 and the second battery 4 continues.

If the power levels of the current power supply battery and the current rechargeable battery meet the fifth preset condition, reselect the battery with higher power level as the power supply battery, and mark the other battery as the rechargeable battery; switch the updated power supply battery to power the main device, control the external energy storage power supply to charge only the updated rechargeable battery, and continue to perform the fourth real-time monitoring of the power levels of the first battery 3 and the second battery 4.

As described above, if the power of the current power supply battery (first battery 3) and the current charging battery (second battery 4) meets the fifth preset condition, the second battery 4 is updated to be marked as a power supply battery, and the first battery 3 is updated to be marked as a charging battery. In conjunction with FIG. 2, at this time, the circuit control unit 103 controls the second MOS switch 10 and the fifth MOS switch 13 to be turned on, and the first MOS switch 9, the third MOS switch 11 and the fourth MOS switch 12 are turned off. At this time, the second battery 4 is switched to supply power to the main device 5, and the external energy storage power supply 2 only charges the first battery 3.

If the power levels of the first battery 3 and the second battery 4 do not satisfy the fourth preset condition, a sixth real-time monitoring is performed on the power levels of the current power supply battery and the current charging battery.

Specifically, in an embodiment of the present invention, the sixth real-time monitoring is implemented using the sixth preset condition, and the sixth preset condition is set to a second preset ratio in which the power value of the current power supply battery is less than the power value of the current charging battery, and the second preset ratio is greater than or equal to 99% and less than or equal to 99.7%.

If the power levels of the current power supply battery and the current charging battery do not meet the sixth preset condition, the fourth real-time monitoring of the power levels of the first battery 3 and the second battery 4 continues.

If the power levels of the current power supply battery and the current rechargeable battery meet the sixth preset condition, the battery with higher power level is reselected and marked as the power supply battery, and the other battery is marked as the rechargeable battery; the updated power supply battery is switched to power the main device 5, the external energy storage power supply 2 is controlled to charge only the updated rechargeable battery, and the fourth real-time monitoring of the power levels of the first battery 3 and the second battery 4 is continued.

As described above, if the power of the current power supply battery (first battery 3) and the current charging battery (second battery 4) meets the sixth preset condition, the second battery 4 is updated to be marked as a power supply battery, and the first battery 3 is updated to be marked as a charging battery. In conjunction with FIG. 2, at this time, the circuit control unit 103 controls the second MOS switch 10 and the fifth MOS switch 13 to be turned on, and the first MOS switch 9, the third MOS switch 11 and the fourth MOS switch 12 are turned off. At this time, the second battery 4 is switched to supply power to the main device 5, and the external energy storage power supply 2 only charges the first battery 3.

When the power of the external energy storage power source 2 is insufficient to charge a single battery, the duty cycle of the corresponding battery charging is reduced to adjust the power.

Further, if the power levels of the first battery 3 and the second battery 4 do not satisfy the fourth preset condition, a seventh real-time monitoring is performed on the external energy storage power supply voltage 2.

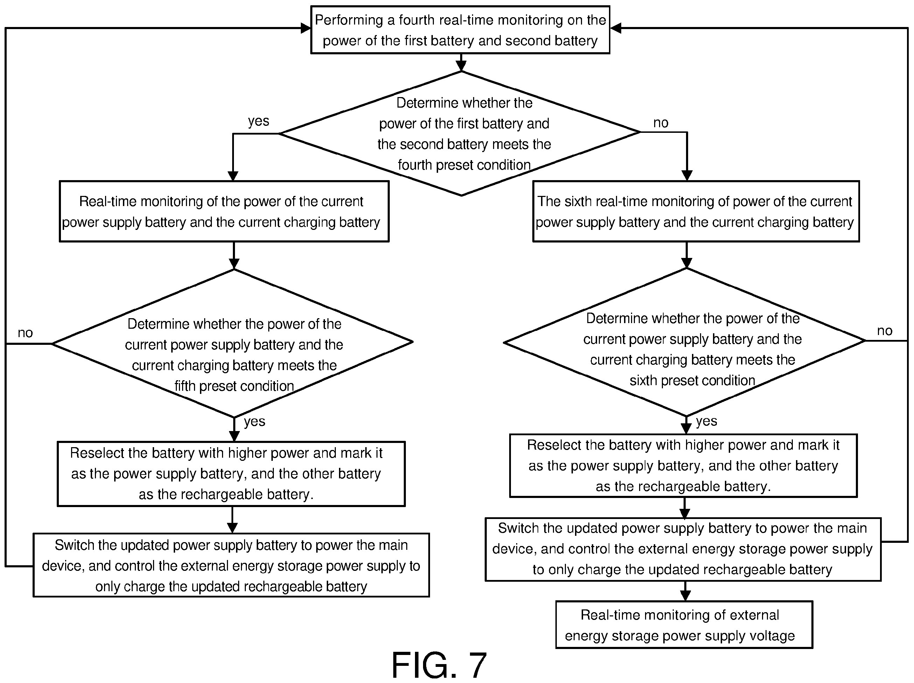

Referring to FIG. 7, preferably, if the power levels of the current power supply battery and the current rechargeable battery meet the sixth preset condition, the circuit control unit 103 selects the battery with higher power level as the power supply battery and marks the other battery as the rechargeable battery; switches the power supply battery to power the main device, and controls the external energy storage power supply 2 to only charge the rechargeable battery, and then performs the seventh real-time monitoring of the external energy storage power supply voltage 2.

Specifically, in the embodiment of the present invention, the seventh real-time monitoring is implemented by using the seventh preset condition, and the seventh preset condition is set as the external energy storage power supply voltage is lower than the fourth preset threshold value for a second consecutive time period, the second time period is greater than or equal to 100 milliseconds and less than or equal to 200 milliseconds, and the fourth preset threshold value is 96%-98% of the normal value of the external energy storage power supply voltage.

If the external energy storage power supply voltage does not meet the seventh preset condition, the seventh real-time monitoring of the external energy storage power supply voltage continues.

If the voltage of the external energy storage power source meets the seventh preset condition, the second charging power duty cycle of the rechargeable battery is reduced.

When the power duty cycle of charging a single battery is reduced to 0, the working mode of an external energy storage power supply management circuit is switched to the third power supply mode. As shown in FIG. 6, the main device 5 is powered by the external energy storage power supply 2, the first battery 3 and the second battery 4.

Specifically, the second charging power duty cycle of the rechargeable battery is monitored in eighth real time. In the embodiment of the present invention, the eighth real-time monitoring is implemented by using an eighth preset condition, and the eighth preset condition is set as the second charging power duty cycle of the rechargeable battery is reduced to 0.

If the second charging power duty cycle of the rechargeable battery does not meet the eighth preset condition, the eighth real-time monitoring of the second charging power duty cycle of the rechargeable battery continues.

If the second charging power duty cycle of the rechargeable battery meets the eighth preset condition, the external energy storage power supply 2 and the first battery 3 and the second battery 4 are controlled to be connected in parallel to supply power to the main device 5.

When the second charging power duty cycle of the rechargeable battery meets the eighth preset condition, the circuit control unit 103 controls the first MOS switch 9, the fourth MOS switch 12 and the fifth MOS switch 13 to be turned on, and the second MOS switch 10 and the third MOS switch 11 to be turned off. At this time, the external energy storage power supply 2, the first battery 3 and the second battery 4 are connected in parallel to supply power to the main device 5.

In the embodiment of the present invention, for example, the first preset condition, the second preset condition, the third preset condition, the fourth preset condition, the fifth preset condition, the sixth preset condition, the seventh preset condition, and the eighth preset condition are respectively set as follows: the first preset condition is that the voltage of the external energy storage power supply is lower than 95% of the normal value of the external energy storage power supply voltage for 500 milliseconds continuously, the second preset condition is that the first charging power duty cycle of the first battery and the second battery is reduced to 0, the third preset condition is that the first power value of the first battery 3 and the second power value of the second battery 4 are both less than 2% of their respective corresponding self-power, the fourth preset condition is that one of the first power value of the first battery 3 and the second power value of the second battery 4 exceeds 8% of their respective corresponding third preset threshold self-power, the fifth preset condition is that the power value of the current power supply battery is less than 92% of the power value of the current charging battery, the sixth preset condition is that the power value of the current power supply battery is less than 99% of the power value of the current charging battery, the seventh preset condition is that the voltage of the external energy storage power supply is lower than 96% of the normal value of the external energy storage power supply voltage for 200 milliseconds continuously, and the eighth preset condition is that the second charging power duty cycle of the charging battery is reduced to 0. In the embodiment of the present invention, for example, the first preset condition, the second preset condition, the third preset condition, the fourth preset condition, the fifth preset condition, the sixth preset condition, the seventh preset condition, and the eighth preset condition are respectively set as follows: the first preset condition is that the voltage of the external energy storage power supply is lower than 90% of the normal value of the external energy storage power supply voltage for 1000 milliseconds continuously, the second preset condition is that the first charging power duty cycle of the first battery and the second battery is reduced to 0, the third preset condition is that the first power value of the first battery 3 and the second power value of the second battery 4 are both less than 5% of their respective corresponding self-power, the fourth preset condition is that one of the first power value of the first battery 3 and the second power value of the second battery 4 exceeds 3% of their respective corresponding third preset threshold self-power, the fifth preset condition is that the power value of the current power supply battery is less than 95% of the power value of the current charging battery, the sixth preset condition is that the power value of the current power supply battery is less than 99.5% of the power value of the current charging battery, the seventh preset condition is that the voltage of the external energy storage power supply is lower than 98% of the normal value of the external energy storage power supply voltage for 100 milliseconds continuously, and the eighth preset condition is that the second charging power duty cycle of the charging battery is reduced to 0.

In the embodiment of the present invention, for example, the first preset condition, the second preset condition, the third preset condition, the fourth preset condition, the fifth preset condition, the sixth preset condition, the seventh preset condition, and the eighth preset condition are respectively set as follows: the first preset condition is that the voltage of the external energy storage power supply is lower than 92% of the normal value of the external energy storage power supply voltage for 800 milliseconds continuously, the second preset condition is that the first charging power duty cycle of the first battery and the second battery is reduced to 0, the third preset condition is that the first power value of the first battery 3 and the second power value of the second battery 4 are both less than 4% of their respective corresponding self-power, the fourth preset condition is that one of the first power value of the first battery 3 and the second power value of the second battery 4 exceeds 5% of their respective corresponding third preset threshold self-power, the fifth preset condition is that the power value of the current power supply battery is less than 97% of the power value of the current charging battery, the sixth preset condition is that the power value of the current power supply battery is less than 99.7% of the power value of the current charging battery, the seventh preset condition is that the voltage of the external energy storage power supply is lower than 97% of the normal value of the external energy storage power supply voltage for 150 milliseconds continuously, and the eighth preset condition is that the second charging power duty cycle of the charging battery is reduced to 0.

In addition, an embodiment of the present invention also provides an external energy storage power supply management device, which includes: a processor and a memory; the memory is used to store one or more program instructions; the processor is used to run one or more program instructions to execute the steps of an external energy storage power supply management method as described in any of the above items.

In addition, an embodiment of the present invention further provides a computer-readable storage medium, on which a computer program is stored. When the computer program is executed by a processor, the steps of an external energy storage power supply management method as described in any of the above items are implemented.

In the embodiment of the present invention, the processor may be an integrated circuit chip having the ability to process signals. The processor may be a general-purpose processor, a digital signal processor (DSP), an application specific integrated circuit (ASIC), a field programmable gate array (FPGA), a programmable gate array (PGA) or other programmable logic devices, discrete gate or transistor logic devices, or discrete hardware components.

The methods, steps and logic block diagrams disclosed in the embodiments of the present invention can be implemented or executed. The general processor can be a microprocessor or the processor can also be any conventional processor, etc. The steps of the method disclosed in the embodiments of the present invention can be directly embodied as a hardware decoding processor for execution, or can be executed by a combination of hardware and software modules in the decoding processor. The software module can be located in a mature storage medium in the field such as a random access memory, a flash memory, a read-only memory, a programmable read-only memory or an electrically erasable programmable memory, a register, etc. The processor reads the information in the storage medium and completes the steps of the above method in combination with its hardware.

The storage medium may be a memory, which may be, for example, a volatile memory or a nonvolatile memory, or may include both volatile and nonvolatile memory.

Among them, the non-volatile memory can be a read-only memory (ROM), a programmable ROM (PROM), an erasable programmable read-only memory (EPROM), an electrically erasable programmable read-only memory (EEPROM), or a flash memory.

The volatile memory may be a random access memory (RAM) which is used as an external cache. By way of example and not limitation, many forms of RAM are available, such as static RAM (SRAM), dynamic RAM (DRAM), synchronous DRAM (SDRAM), double data rate synchronous dynamic random access memory (DDRSDRAM), enhanced synchronous dynamic random access memory (ESDRAM), Synchlink DRAM (SLDRAM), and direct RAM bus random access memory (DRRAM).

The storage media described in the embodiments of the present invention is intended to include, but are not limited to, these and any other suitable types of memory.

Those skilled in the art will appreciate that in one or more of the above examples, the functions described in the present invention may be implemented using a combination of hardware and software. When software is used, the corresponding functions may be stored in a computer-readable medium or transmitted as one or more instructions or codes on a computer-readable medium. Computer-readable media include computer storage media and communication media, wherein communication media include any media that facilitates the transmission of computer programs from one place to another. Storage media may be any available media that can be accessed by a general or special purpose computer.

Although the present invention has been described in detail above by general description and specific embodiments, it is obvious to those skilled in the art that some modifications or improvements can be made on the basis of the present invention. Therefore, these modifications or improvements made on the basis of not departing from the spirit of the present invention all belong to the scope of protection claimed by the present invention.

## Claims

1. A method for managing an external energy storage power supply, **characterized in that** the method is applied to a power management module, comprising:
performing a first real-time monitoring of the voltage of the external energy storage power supply;
controlling the external energy storage power supply to directly power the main device, and charging the first battery and the second battery at the same time, and monitoring, continuously, the voltage of the external energy storage power supply in a first real-time manner, in response to the voltage of the external energy storage power supply not meeting the first preset condition;
lowering the first charging power duty cycle of the first battery and the second battery in response to the voltage of the external energy storage power supply meeting the first preset condition;
performing a second real-time monitoring on the first charging power duty cycle of the first battery and the second battery;
continuing to perform the first real-time monitoring of the external energy storage power supply voltage in response to the first charging power duty ratio of the first battery and the second battery not meeting the second preset condition;
performing a third real-time monitoring on the power levels of the first battery and the second battery in response to the first charging power duty ratios of the first battery and the second battery meeting the second preset condition;
controlling the external energy storage power supply and the first battery and the second battery to be connected in parallel to supply power to the main device in response to the power levels of the first battery and the second battery meeting a third preset condition;
selecting and marking the battery with higher power level as the power supply battery, and marking the other battery as the rechargeable battery in response to the power levels of the first battery and the second battery not meeting the third preset condition; and
switching the power supply battery to supply power to the main device, and controlling the external energy storage power supply to only charge the rechargeable battery.

2. An external energy storage power supply management method as described in claim 1, **characterized in that** the first preset condition is set as the voltage of the external energy storage power supply being lower than a first preset threshold value for a continuous first time period, the first time period is 500 milliseconds-1000 milliseconds, and the first preset threshold value is 90% to 95% of the normal value of the external energy storage power supply voltage.

3. The external energy storage power management method according to claim 2, wherein the second preset condition is set as the first charging power duty cycle of the first battery and the second battery is reduced to 0.

4. A method for managing an external energy storage power supply as claimed in claim 3, **characterized in that** the third preset condition is set as the first power value of the first battery and the second power value of the second battery have not reached their respective corresponding second preset thresholds, and the second preset thresholds corresponding to the first battery and the second battery are 2% to 5% of their own power.

5. A method for managing an external energy storage power supply as claimed in claim 4, **characterized in that** the method further includes:
performing a fourth real-time monitoring on the power levels of the first battery and the second battery in response to power levels of the first battery and the second battery not meeting the third preset condition;
performing a fifth real-time monitoring on the power levels of the current power supply battery and the current charging battery in response to the power levels of the first battery and the second battery meeting a fourth preset condition;
continuing to perform fourth real-time monitoring on the power levels of the first battery and the second battery in response to the power levels of the current power supply battery and the current charging battery not meeting the fifth preset condition;
reselecting the battery with higher power level as the power supply battery and marking the other battery as the rechargeable battery in response to the power levels of the current power supply battery and the current rechargeable battery meeting the fifth preset condition;
switching the updated power supply battery to power the main device, controlling the external energy storage power supply to charge only the updated rechargeable battery, and continuing to perform a fourth real-time monitoring of the power of the first battery and the second battery;
wherein the fourth preset condition is set as one of the first power value of the first battery and the second power value of the second battery exceeds their respective corresponding third preset thresholds, and the third preset thresholds corresponding to the first battery and the second battery are respectively greater than or equal to 3% to 8% of their own power; the fifth preset condition is set as the power value of the current power supply battery is less than a first preset ratio of the power value of the current charging battery, and the first preset ratio is greater than or equal to 92% and less than or equal to 97%.

6. The external energy storage power management method according to claim 5, **characterized in that** the method further comprises:
performing a sixth real-time monitoring on the power levels of the current power supply battery and the current charging battery in response to the power levels of the first battery and the second battery not meeting the fourth preset condition;
continuing to perform fourth real-time monitoring on the power levels of the first battery and the second battery in response to the power levels of the current power supply battery and the current charging battery not meeting the sixth preset condition;
reselecting the battery with higher power level as the power supply battery and marking the other battery as the rechargeable battery in response to the power levels of the current power supply battery and the current rechargeable battery meeting the sixth preset condition;
switching the updated power supply battery to power the main device, controlling the external energy storage power supply to charge only the updated rechargeable battery, and continuing to perform a fourth real-time monitoring of the power of the first battery and the second battery;
setting the sixth preset condition as a second preset ratio that the power value of the current power supply battery is less than the power value of the current charging battery, wherein the second preset ratio is greater than or equal to 99% and less than or equal to 99.7%.

7. The external energy storage power management method according to claim 6, **characterized in that** the method further comprises:
performing a seventh real-time monitoring on the voltage of the external energy storage power supply in response to the power levels of the first battery and the second battery not meeting the fourth preset condition;
continuing to perform seventh real-time monitoring on the voltage of the external energy storage power supply in response to the voltage of the external energy storage power supply not meeting the seventh preset condition;
lowering the second charging power duty cycle of the rechargeable battery in response to the voltage of the external energy storage power supply meeting the seventh preset condition;
performing an eighth real-time monitoring on the second charging power duty cycle of the rechargeable battery;
continuing to perform an eighth real-time monitoring on the second charging power duty cycle of the rechargeable battery in response to the second charging power duty cycle of the rechargeable battery not meeting the eighth preset condition;
controlling the external energy storage power supply and the first battery and the second battery to be connected in parallel to supply power to the main device in response to the second charging power duty cycle of the rechargeable battery meeting the eighth preset condition;
wherein the seventh preset condition is set as the voltage of the external energy storage power supply is lower than the fourth preset threshold for a second consecutive time period, the second time period is 100 to 200 milliseconds, and the first preset threshold is 96% to 98% of the normal value of the external power supply voltage ; the eighth preset condition is set as the second charging power duty cycle of the rechargeable battery is reduced to 0.

8. An external energy storage power management device, **characterized in that** the device comprises:
a memory that stores one or more program instructions; and
a processor connected to the memory, the processor being configured to run the one or more program instructions to execute the steps of an external energy storage power supply management method as described in claim 1.

9. A computer-readable storage medium, **characterized in that** a computer program is stored on the computer-readable storage medium, and when the computer program is executed by a processor, the steps of the external energy storage power supply management method according to claim 1 are implemented.

10. An external energy storage power supply management system, **characterized in that** the system is applied to a power management module, and the system comprises:
an external energy storage power supply voltage monitoring unit that is configured to perform a first real-time monitoring on the external energy storage power supply voltage;
a battery monitoring unit that is configured to perform a second real-time monitoring on the first charging power duty cycle of the first battery and the second battery; and
a circuit control unit;
wherein the external energy storage power supply management system is configured to:
control, by the circuit control unit, the external energy storage power supply to directly power the main device and charge the first battery and the second battery at the same time, and continue, by the external energy storage power supply voltage monitoring unit, to perform first real-time monitoring on the voltage of the external energy storage power supply in response to the voltage of the external energy storage power supply not meeting the first preset condition;
lower, by the circuit control unit, the first charging power duty cycle of the first battery and the second battery in response to the voltage of the external energy storage power supply meeting the first preset condition;
continue, by the battery monitoring unit, to perform first real-time monitoring on the voltage of the external energy storage power supply in response to the first charging power duty ratio of the first battery and the second battery not meeting the second preset condition;
perform, by the battery monitoring unit a third real-time monitoring on the power levels of the first battery and the second battery in response to the first charging power duty ratios of the first battery and the second battery meeting the second preset condition;
control, by the circuit control unit, the external energy storage power supply and the first battery and the second battery to be connected in parallel to supply power to the main device in response to the power levels of the first battery and the second battery meeting the third preset condition;
select, by the circuit control unit, the battery with higher power level as the power supply battery, mark, by the circuit control unit, the other battery as the rechargeable battery, switch, by the circuit control unit, the power supply battery to power the main device, and control, by the circuit control unit, the external energy storage power supply to only charge the rechargeable battery in response to the power levels of the first battery and the second battery not meeting the third preset condition.
